# EUROPEAN PATENT SPECIFICATION

(11) **EP 0 486 461 B1**
(45) Date of publication and mention of the grant of the patent: **17.01.1996**
(21) Application number: 91890268.5
(22) Date of filing: 06.11.1991
(51) Int. Cl.: C07D 405/14, C07D 307/52

(54) **A diketene imine derivative and process for preparing same**
Diketeniminderivat und Verfahren zu seiner Herstellung
Dérivé de dicétène imine et procédé pour leur préparation

(30) Priority: 09.11.1990 HU 707390
(43) Date of publication of application: 20.05.1992
(73) Proprietor: RICHTER GEDEON VEGYESZETI GYAR R.T., H-1475 Budapest (HU)
(72) Inventor: Jeszenski, Andor, Dipl.-Chem., H-1025 Budapest (HU); Losonczi, Béla, Dr., Dipl.-Chem., H-1124 Budapest (HU); Czéh, Mária, H-2521 Csolnok (HU); Godó, László, H-1161 Budapest (HU); Stefkó, Béla, dr., H-1111 Budapest (HU); Benczik, Tamás, Dipl.-Chem. Ing., H-2500 Esztergom (HU); Hermann, Lázsló, Dipl.-Chem Ing., H-1183 Budapest (HU); Havasi, Gábor, Dipl.-Chem Ing., H-2510 Dorog (HU); Kálvin, Péter, Dipl.-Chem Ing., H-2509 Esztergom-Kertváros (HU); Stefán, László, Dipl.-Chem Ing., H-2510 Dorog (HU); Megyeri, Gábor, Dr. Dipl.-Chem Ing., H-1021 Budapest (HU)
(74) Representative: Puchberger, Rolf, Dipl. Ing.

(56) References cited:
- EP-A- 0 269 315
- DD-A- 209 830
- DE-A- 2 734 070
- GB-A- 2 169 600

## Description

GB - A- 2 169 600, DE-A- 2 734 070, EP-A- 0 269 315 and DD- A - 209 830 relate to processes for the preparation of ranitidine and intermediates thereof.

This invention relates to a diketene imine derivative of the formula (I) 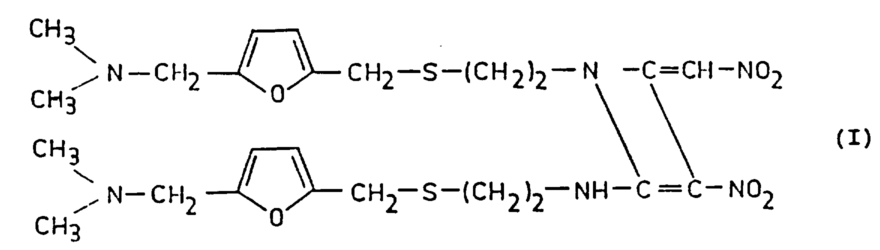 chemically 1-{2-[(5-N,N-dimethylaminomethyl-2-furyl)-methylthio]ethyl}-2-{2-[(5-N,N-dimethylaminomethyl-2-furyl)-methylthio]-1-ethylamino}-3-nitro-4-nitromethylene-2-azetine (hereinafter abbreviated: diketene imine compound) as well as to the preparation process of this compound.

The diketene imine compound of formula (I) of the invention is till now unknown in the literature. The compound of formula (I) is a valuable intermediate in the synthesis of 1-{2-[(5-dimethylaminomethyl-2-furyl)methylthio]ethyl}-amino-1-methylamino-2-nitroethylene (generic name: ranitidine), a known H-2 receptor antagonist.

The aim of the present invention is to provide for the synthesis of the above-mentioned H-2 receptor-blocking ranitidine a key intermediate, which can be produced by a single technological process-step being conveniently practicable on an industrial scale and which, on the other hand, can lead to the aimed H-2 receptor antagonist in one simple step.

It has been recognized during our investigations that this aim can easily be achieved in such a way that, after reacting the furfuryl derivative of formula (II) 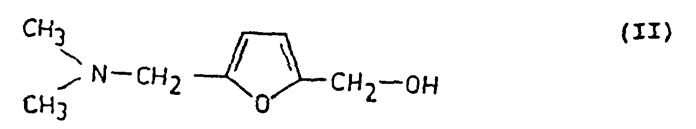 with cysteamine hydrochloride of the formula (III)

HS-CH₂-CH₂-NH2 · HCl (III)

then with 1-nitro-2,2-di(methylthio)ethene of the formula (IV) 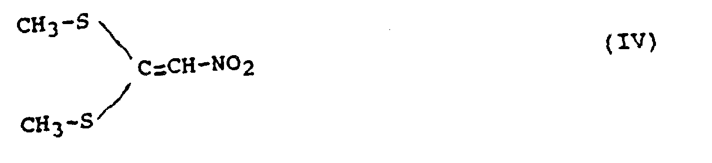 and subsequently removing the unreacted nitro compound, the mixture is reacted with a zinc salt dissolved in an alkanol. In this way the diketene imine compound of formula (I) is obtained.

The compound of formula (I) can simply be transformed to ranitidine in a single step in a yield of nearly 100%.

Thus, the invention further relates to a process for the preparation of the compound of formula (I), which comprises reacting the furfuryl alcohol derivative of formula (II) with cysteamine hydrochloride of the formula (III), then with an inorganic base and subsequently with the nitro compound of formula (IV) and, after removal of the unreacted nitro compound of formula (IV) from the reaction mixture, adding a zinc salt to the residue in a C₁₋₄alkanol medium in the presence of a base.

According to a preferred embodiment of the process of the invention zinc chloride or zinc sulfate is used as a zinc salt.

According to an other embodiment of the process of the invention, the reaction with the zinc salt is carried out in the presence of an organic base, suitable a tertiary amine; triethylamine is preferably used as a tertiary amine.

The advantage of the process according to the invention lies therein that the valuable key intermediate of formula (I) can be prepared by using a simple technological procedure which is prominently utilizable on industrial scales.

The structure of the compound of formula (I) was determined by the means of IV, IR and NMR spectroscopy.

The invention is illustrated in detail by the following Example.

### EXAMPLE

87 g (0.56 mol) of 4-dimethylaminomethylfurfuryl alcohol of the formula (II) and 51.8 g (0.46 mol) of cysteamine hydrochloride of the formula (III) are portionwise added to 36 g of concentrated aqueous hydrochloric acid, then the reaction mixture is stirred at room temperature for 48 hours. After adding 92 ml of ethanol, the mixture is heated to the boiling point, then cooled down again to room temperature (25°C) and 132 g of 20 % aqueous sodium hydroxide solution are added to the same temperature.

The oily brown product obtained is extracted into 270 ml of toluene. After separation the aqueous phase is washed with 80 ml of toluene, the combined toluene solution is dried over 15 g of anhydrous sodium sulfate and then clarified by 9 g of a mixture containing celite and activated carbon in an 1:1 ratio.

After filtration 61.8 g (0.374 mol) of 1-nitro-2,2-di(methylthio)ethene of the formula (IV) (about the half of which can be recovered from the reaction mixture in a reutilizable form) are added, the raction mixture is refluxed for 1 hour, then cooled to room temperature and extracted twice with 150 ml and then once with 160 ml of 0.1 N hydrochloric acid each. The complete removal by extraction of the unreacted nitro compound is examined by thin layer chromatography (TLC). When the reaction is incomplete, a further extraction is carried out with additional 20 ml of 0.1 N hydrochlorid acid solution. After adjusting the pH value of the hydrochlorid acid solution between 7.5 and 8 by adding 0.2 N aqueous sodium hydroxide solution, 23.5 g of 1-nitro-2,2-di(methylthio)ethene of the formula (IV) can be recovered. The toluene solution is extracted with 400 ml of methanol.

Thereafter, 200 g of zinc chloride are dissolved in 2000 ml of methanol and the above methanolic extract of 400 ml is added dropwise to this solution at room temperature. After adding 100 g of triethylamine, the reaction mixture is stirred at 40°C for 18 hours.

Subsequently, the mixture is clarified with 25 g of activated carbon and 25 g of celite for 30 minutes, then filtered and the solution obtained is evaporated at 20°C to the one third of its original volume under nitrogen under reduced pressure of 10⁻⁴ MPa and then cooled to -15°C. After filtering off the solid precipitate, the oily residue is maintained unter a reduced pressure of 100 Pa until its weight remains unchanged after 2 hours. In this way 50 g of product are obtained (the yield calculated for the nitro compound is 79.5 %), m.p.: from -39°C to -35°C (decomposes over 176°C).
UV (λ max): 331.6 nm
IR: 1370 and 1530 cm⁻¹ (NO₂ group): 1630 cm⁻¹ ( >C=C<)
¹H-NMR (δ, ppm):
2.25 (s, 12H, two 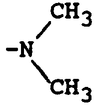 groups)
2.75 (t, 4H, two -S-CH₂-CH₂- groups)
3.53 (s, 4H, two -CH₂-S- groups)
3.60 (m, 4H, two -S-CH₂-CH₂-N- groups)
3.80 (s, 4H, two -N-CH₂- groups)
6.20 (s, 4H, two olefines)
6.80 (s, 4H, >C=CH-NO₂ group)

## Claims

1. The compound 1-{2-[(5-N,N-dimethylaminomethyl-2-furly)-methylthio]ethyl}-2-{2-[(5-N,N-dimethylaminomethyl-2-furyl)methylthio]-1-ethylamino}-3-nitro-4-nitromethylene-2-azetine of the formula (I)

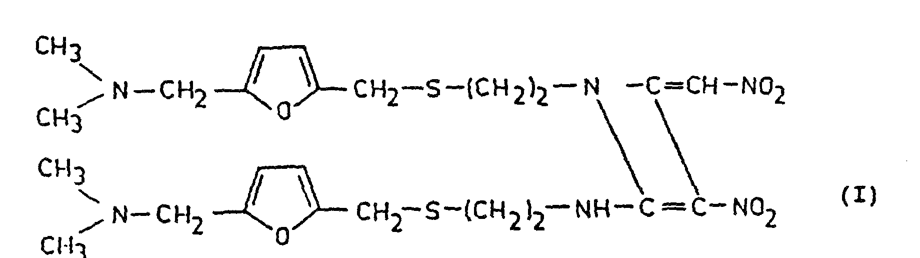

2. A process for the preparation of 1-{2-[(5-N,N-dimethylaminomethyl-2-furyl)-methylthio]ethyl}-2-{2-[(5-N,N-dimethylaminomethyl-2-furyl)methylthio]-1-ethylamino}-3-nitro-4-nitromethylene-2-azetine of the formula (I) 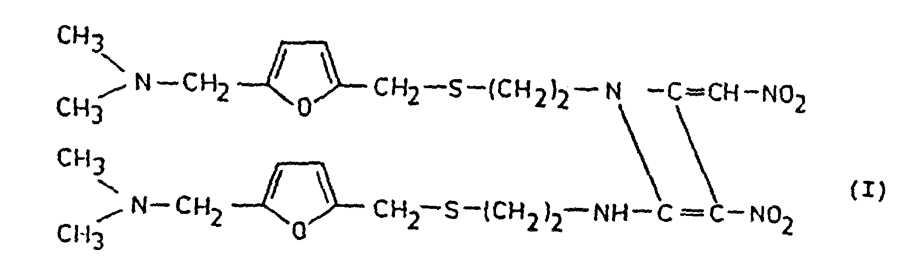 which comprises reacting the furfuryl alcohol derivative of formula (II) 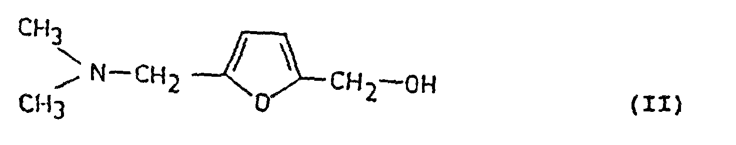 with cysteamine hydrochloride of the formula (III)
HS-CH₂-CH₂-NH2 · HCl (III)
then with an inorganic base, subsequently with 1-nitro-2,2-di(methylthio)ethene of the formula (IV) 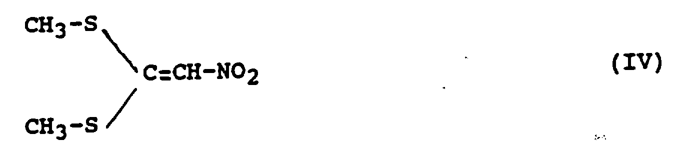 removing the unreacted nitro compound of formula (IV) from the reaction mixture and adding a zinc salt to the residue in an C₁₋₄alkanol medium in the presence of a base.

3. The process as claimed in claim 2, which comprises using zinc chloride or zinc sulfate as a zinc salt.

4. The process as claimed in claim 2 or 3, which comprises carrying out the reaction with the zinc salt in the presence of an organic base, preferably a tertiary amine.

5. The process as claimed in any of the claims 2 to 4, which comprises using triethylamine as tertiary amine.

## Patentansprüche

1. Die Verbindung 1-{2-[(5-N,N-Dimethylaminomethyl-2-furyl)-methylthio]ethyl}-2-{2-[(5-N,N-dimethylaminomethyl-2-furyl)-methylthio]-1-ethylamino} -3-nitro-4-nitromethylen-2-azetin der Formel (I)

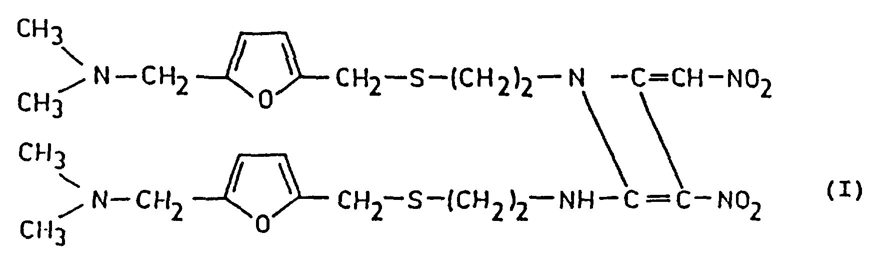

2. Ein Verfahren zur Herstellung von 1-{2-[(5-N,N-Dimethylaminomethyl-2-furyl)-methylthio]ethyl}-2-{2-[(5-N,N-dimethylaminomethyl-2-furyl)methylthio]-1-ethylamino}-3-nitro-4-nitromethylen-2-azetin der Formel (I) 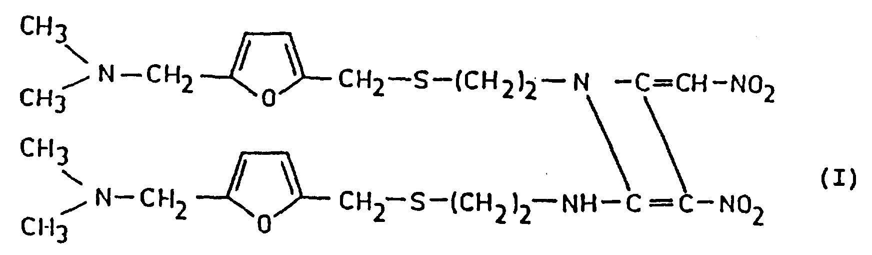 welches Verfahren die Reaktion von Furfurylakoholderivat der Formel (II) 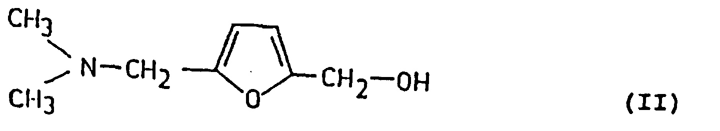 mit Cysteaminhydrochlorid der Formel (III)
HS-CH₂-CH₂-NH2 . HCl (III)
dann mit einer anorganischen Base, darauffolgend mit 1-Nitro-2,2-di(methylthio)ethen der Formel (IV) 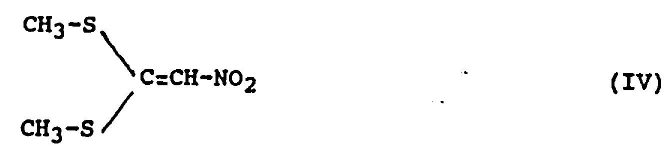 das Entfernen der nichtreagierten Nitroverbindung der Formel (IV) aus der Reaktionsmischung und das Zusetzen von Zinksalz zum Rückstand in einem C₁₋₄-Alkanolmedium bei Vorhandensein einer Base enthält.

3. Verfahren nach Anspruch 2, welches die Verwendung von Zinkchlorid oder Zinksulfat als Zinksalz enthält.

4. Verfahren nach Anspruch 2 oder 3, welches das Durchführen der Reaktion mit dem Zinksalz bei Vorhandensein einer organischen Base, vorzugsweise einem tertiären Amin, enthält.

5. Verfahren nach einem der Ansprüche 2 bis 4, welches die Verwendung von Triethylamin als tertiäres Amin enthält.

## Revendications

1. Composé de type 1-{2-[(5-N, N-diméthylaminométhyl-2-furyl)-méthylthio]éthyl}-2-{2-[(5-N,N-diméthylaminométhyl-2-furyl)-méthylthio]-1-éthylamino}-3-nitro-4-nitrométhyléne-2-azétine répondant à la formule (I):

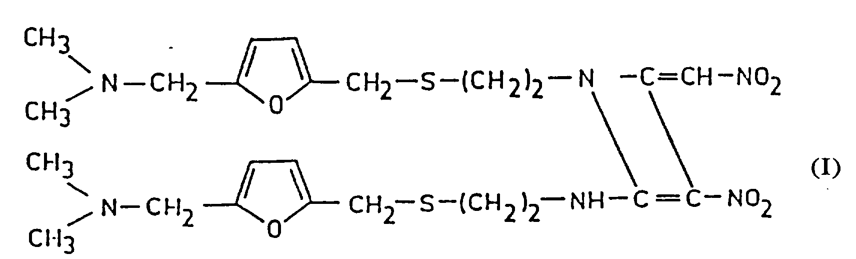

2. Procédé pour la préparation du 1-{2-[(5-N,N-diméthylaminométhyl-2-furyl)-méthylthio]éthyl}-2-{2-[(5-N,N-diméthylarninométhyl-2-furyl)-méthylthio]-1-éthylamino}-3-nitro-4-nitrométhylène-2-azétine répondant à la formule (I): 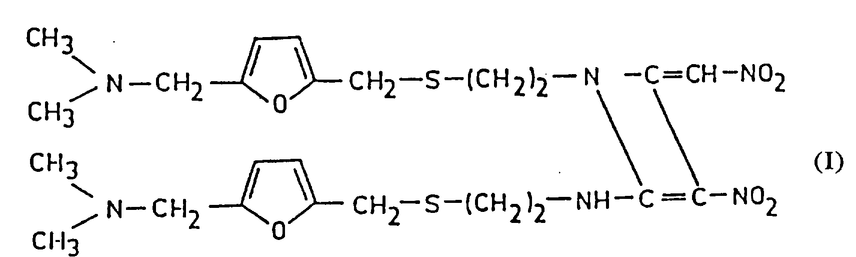 comportant le fait de faire réagir un dérivé de l'alcool furfurylique répondant à la formule (II) : 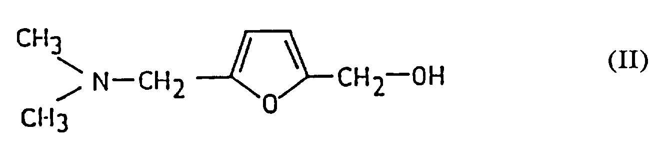 avec de l'hydrochlorure de cystéamine répondant à la formule (III) :
HS-CH₂-CH₂-NH₂ . HCl (III)
puis avec une base inorganique, suivie du 1-nitro-2,2-di(méthyl-thio)éthène répondant à la formule (IV) 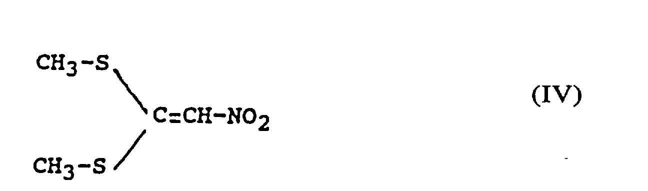 d'éliminer du milieu réactionnel le composé porteur d'un groupe nitro de formule (IV) n'ayant pas réagi et d'ajouter au résidu un sel de zinc, dans un milieu constitué d'alcanol en C₁₋₄ en présence d'une base.

3. Procédé conforme à la revendication 2, dans lequel le sel de zinc utilisé est un chlorure de zinc ou un sulfate de zinc.

4. Procédé conforme à la revendication 2 ou 3, dans lequel la réaction est effectuée avec un sel de zinc en présence d'une base organique, de préférence une amine tertiaire.

5. Procédé conforme à une quelconque des revendications 2 à 4, qui consiste à utiliser la triéthylamine comme amine tertiaire.
